(19) Europäisches Patentamt
European Patent Office
Office européen des brevets

(11) Veröffentlichungsnummer: **0 031 537**
**B1**

# (12) EUROPÄISCHE PATENTSCHRIFT

(45) Veröffentlichungstag der Patentschrift: **18.05.83**

(51) Int. Cl.³: **C 07 D 301/12**, C 07 D 303/04

(21) Anmeldenummer: **80107933.6**

(22) Anmeldetag: **16.12.80**

(54) **Verfahren zur Herstellung von Oxiranen.**

(30) Priorität: **29.12.79 DE 2952755**

(43) Veröffentlichungstag der Anmeldung: **08.07.81 Patentblatt 81/27**

(45) Bekanntmachung des Hinweises auf die Patenterteilung: **18.05.83 Patentblatt 83/20**

(84) Benannte Vertragsstaaten: **AT BE CH DE FR GB IT LI NL**

(56) Entgegenhaltungen:
**DE-A 2 752 626**
**DE-A 2 803 791**
**DE-A 2 916 834**
**Patents Abstracts of Japan Band 3, Nr. 14, 8. Februar 1979 Seite 40C36**
**Patents Abstracts of Japan Band 4, Nr. 22, 23. Februar 1980 Seite 46C74**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Pohl, Fritz, Dr., Karl-Marx-Strasse 5, D-3300 Braunschweig (DE)**
Erfinder: **Rauleder, Gebhard, Dr., Mozartstrasse 20, D-5657 Haan (DE)**
Erfinder: **Waldmann, Helmut, Dr., Henry-T.-von-Böttinger-Strasse 15, D-5090 Leverkusen 1 (DE)**

**EP 0 031 537 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Oxiranen

Die vorliegende Erfindung betrifft ein Verfahren zur Herstellung von Oxiranen aus Olefinen und Wasserstoffperoxid in Gegenwart eines Katalysators auf Basis Bor.

Oxirane finden Verwendung auf dem Gebiet der Lacke, zur Herstellung von Polyethern, Polyurethanen, Epoxidharzen, Detergentien, Glykolen und einer Vielzahl von organischen Zwischenprodukten (s. beispielsweise US-PS 2 412 136).

Es ist bekannt, dass man Oxirane nach der Chlorhydrinmethode durch Umsetzung von Olefinen mit Chlor im alkalischen Medium und nachfolgender Behandlung mit Basen herstellen kann. Ein grundsätzlicher Nachteil dieses Verfahrens besteht darin, dass salzhaltige umweltbelastende Abwässer und unerwünschte chlorierte Nebenprodukte gebildet werden (s. Ullmann's Enzyklopädie der Technischen Chemie, Band 10, Seite 565 (3. Auflage).

Ethylenoxid, eine wichtige Verbindung aus der Gruppe der Oxirane, wird technisch durch Oxidation von Ethylen mit molekularem Sauerstoff in der Gasphase in Gegenwart von Silberkatalysatoren hergestellt (s. beispielsweise US-PS 2 693 474). Eine Übertragung dieser Methode auf höhere Olefine scheiterte bislang, da dann in grossem Ausmass Nebenreaktionen ablaufen, welche die Wirtschaftlichkeit eines solchen Verfahrens entscheidend vermindern (s. US-PS 2 412 136).

Ein weiteres Syntheseprinzip beruht auf der Umsetzung von Olefinen mit organischen Hydroperoxiden in Gegenwart von Katalysatoren (s. DE-AS 14 68 012). Diese Verfahren haben den entscheidenden Nachteil, dass das organische Hydroperoxid ROOH, wobei R z.B. einen niedermolekularen Rest, wie t-Butyl oder Cumyl bedeuten kann, gemäss nachstehender Gleichung während der Reaktion in den entsprechenden Alkohol ROH umgewandelt wird:

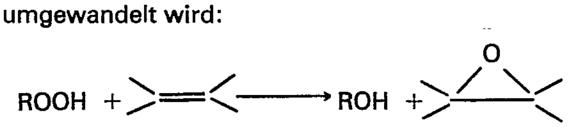


Es fällt somit der dem eingesetzten Hydroperoxid entsprechende Alkohol als Koppelprodukt an, was eine Abhängigkeit der Wirtschaftlichkeit dieses Verfahrens von der Verwertbarkeit dieses Alkohols mit sich bringt. Falls er nicht verwertet werden kann, muss er über mehrere Verfahrensstufen in das entsprechende Hydroperoxid zurückverwandelt werden, was besonderen technischen Aufwand erfordert.

Weiterhin ist es bekannt, Olefine mit einer Percarbonsäure in die entsprechenden Oxirane umzuwandeln (s. N. Prileschajew, Ber. dtsch. chem. Ges. 42, 4811 (1909)). Die Percarbonsäuren lassen sich in einer Gleichgewichtsreaktion aus Wasserstoffperoxid und einer Carbonsäure in Gegenwart von starken Säuren, wie Schwefelsäure, herstellen (s. S. N. Lewis in R. L. Augustin «Oxidation», Vol. 1, S. 216, Marcel Dekker Verlag, New York (1969)).

Bei der Umsetzung der dabei erhaltenen Percarbonsäure mit Olefinen können Oxiranverluste auftreten, da die im Reaktionsgemisch vorhandene Mineralsäure die Aufspaltung des primär gebildeten Oxirans bewirken kann (D. Swen «Organic Peroxides», Vol. 2, S. 436, Wiley Interscience (1971)).

Eine weitere Methode zur Herstellung von Oxiranen beruht auf der Umsetzung von Olefinen mit Wasserstoffperoxid in Gegenwart von Katalysatoren. So wird z.B. in der BE-PS 838 953 die Verwendung von Arsenverbindungen als Katalysatoren beschrieben. Die Anwendung von Arsen als Katalysator stellt jedoch wegen der hohen Toxizität dieses Elements ein Problem dar und gleichzeitig besteht die Notwendigkeit, zur Erzielung akzeptabler Ausbeuten sehr hochkonzentriertes Wasserstoffperoxid zu verwenden (s. DE-OS 29 16 834).

Ein anderes Katalysatorsystem wird in der DE-OS 20 12 049 beschrieben. Gemäss Beispiel 58 wurde Cyclohexen mit Wasserstoffperoxid in Gegenwart einer Zinn- und einer Borverbindung als Katalysator umgesetzt. Dabei wurde bei einem Wasserstoffperoxid-Umsatz von etwa 12% Cyclohexenoxid mit einer Selektivität von 65% erhalten. Das ist für eine technische Anwendung völlig unbefriedigend.

Eine Verbesserung dieser Methode konnte dadurch erreicht werden, dass man das mit $H_2O_2$ eingebrachte und während der Reaktion gebildete Wasser kontinuierlich aus dem Reaktionsgemisch entfernte.

So wird in der DE-OS 27 52 626 und der DE-OS 28 03 791 die Umsetzung von Olefinen mit Wasserstoffperoxid in Gegenwart von Übergangsmetallverbindungen, Arsenverbindungen oder Borverbindungen als Katalysatoren beschrieben, wobei die Reaktion zwischen 0 und 120 °C durchgeführt und das im Verlauf der Reaktion gebildete und mit dem Wasserstoffperoxid eingeführte Wasser kontinuierlich aus dem Reaktionsgemisch entfernt wird.

Ein besonderer Nachteil dieses Verfahrens besteht darin, dass Wasser nur dann während der Reaktion durch Destillation entfernt werden kann, wenn der Siedepunkt des eingesetzten Olefins über dem Siedepunkt des Wassers liegt. Dadurch ist dieses Verfahren ungeeignet zur Herstellung so wichtiger Epoxide wie z.B. Ethylenoxid oder Propylenoxid. In Fällen, in denen das Wasser mit dem Olefin ein Azeotrop bildet oder zur azeotropen Entfernung des Wassers ein geeignetes zusätzliches Lösungsmittel eingesetzt wird, kann die Temperatur für die Reaktion nicht mehr frei gewählt werden, da diese durch den Siedepunkt des Azeotrops festgelegt ist. In den Fällen, in denen das Wasser durch den Epoxidationskatalysator gebunden wird, sind grosse Katalysatormengen und eine technisch aufwendige Regeneration des Katalysators erforderlich.

Eine Vereinfachung dieser verfahrenstechnisch aufwendigen Methode, die zur Herstellung von

technisch so wichtigen Oxiranen wie Ethylenoxid und Propylenoxid ungeeignet ist, wird in der DE-OS 29 16 834 beschrieben. Dabei werden Olefine mit Wasserstoffperoxid in Gegenwart von Borverbindungen bei einer Reaktionstemperatur von 120 bis 200 °C ungesetzt, ohne das Reaktionswasser abzudestillieren. Ein grundsätzlicher Nachteil dieses Verfahrens besteht in der Notwendigkeit, hohe Temperaturen im Bereich von 120 bis 200 °C anwenden zu müssen. In diesem Temperaturbereich befindet sich Wasserstoffperoxid in einem metastabilen Zustand, wobei die Zersetzungsgeschwindigkeit exponentiell mit steigender Reaktionstemperatur zunimmt. Das wirft grosse sicherheitstechnische Probleme auf. Wie aus der DE-OS 29 16 834, Seite 11, Zeile 5 ff. hervorgeht, muss zur Stabilisierung des Wasserstoffperoxids im sauren Milieu gearbeitet werden. Dabei tritt aber als grosser Nachteil eine Aufspaltung der Oxirane auf, was nur dadurch eingeschränkt werden kann, dass man noch Puffersubstanzen, wie Amine, zur Reaktionsmischung gibt. Die Amine können ihrerseits von Wasserstoffperoxid ebenfalls oxidiert werden. Weiterhin ist das Problem der Aufarbeitung des Reaktionsgemisches, der Isolierung des Oxirans und der Rückführung von nicht-umgesetzten Wasserstoffperoxid und Olefin und Kreislaufführung des verwendenten Lösungsmittels noch nicht gelöst. Die Lösung dieser Probleme ist aber notwendig, um ein technisch und wirtschaftlich brauchbares Verfahren zur Verfügung zu haben.

Schliesslich wird in der JP-OS 53–137903 ein Verfahren zur Herstellung von Propylenoxid aus Propylen und Wasserstoffperoxid beschrieben, bei dem jedoch keine Angaben zur Isolierung des Propylenoxids und zur Abtrennung und Wiederverwendung von nicht umgesetztem Propylen und Katalysator gemacht werden. Gute Ausbeuten an Propylenoxid werden nur beim Einsatz hochkonzentrierter Wasserstoffperoxidlösungen erhalten, was enorme sicherheitstechnische Aufwendungen erfordert. Ausserdem sind solche hochkonzentrierten Wasserstoffperoxidlösungen nur zusammen mit Wasser möglich, was bedeutet, dass bei diesem Verfahren vergleichsweise viel Wasser zugegen ist. Wenn in dieses Verfahren Borsäure als Katalysator eingesetzt wird, so beträgt die Selektivität der Propylenoxid-Bildung nur 38,8%.

Es wurde nun überraschenderweise gefunden, dass man Oxirane aus Olefinen und Wasserstoffperoxid in Gegenwart von Orthoborsäure herstellen kann, wenn man

a) ein Olefin der allgemeinen Formel

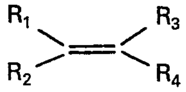

(I),

worin $R_1$, $R_2$, $R_3$ und $R_4$ unabhängig voneinander Wasserstoff, $C_1$– bis $C_{30}$– Alkyl, $C_1$– bis $C_{15}$– Alkenyl, $C_3$– bis $C_{12}$– Cycloalkyl, Phenyl oder Naphthyl bedeuten und/oder

worin $R_1$ und $R_2$ durch eine Kohlenstoffkette von bis zu 12 Kohlenstoffatomen miteinander verbunden sein können und/oder

worin $R_1$ und $R_3$ zusammen mit der zu epoxidierenden Doppelbindung ein 1– bis 3–cyclisches Ringsystem mit bis zu 12 Kohlenstoffatomen bilden können und worin $R_1$, $R_2$, $R_3$ und $R_4$ gegebenenfalls als Substituenten $C_1$– bis $C_4$–Alkyl, $C_3$– bis $C_6$–Cycloalkyl, $C_1$– bis $C_4$– Alkenyl, Phenyl, $C_1$– bis $C_4$– Alkoxy, Hydroxy, Carboxy oder Halogen enthalten können, wobei zwischen dem Kohlenstoffatom, das als Substituenten Alkoxy, Hydroxy, Carboxy oder Halogen enthält, und der zu epoxidierenden Doppelbindung mindestens noch ein Kohlenstoffatom dazwischen steht,

mit einer weniger als 0,1 Gew.-% Wasser enthaltenden Lösung von Wasserstoffperoxid in einem Lösungsmittel bei einer Temperatur von 30 bis 110 °C und bei einer Reaktionszeit, die je nach Reaktivität des eingesetzten Olefins zwischen 15 Minuten und 5 Stunden beträgt, umsetzt,

b) das entstandene Oxiran und das nicht umgesetzte Olefin abtrennt,

c) vor, während und/oder nach der Abtrennung von Oxiran und Olefin der Reaktionsmischung die Menge Wasserstoffperoxid zusetzt, die bei der Reaktion verbraucht worden ist,

d) nach Durchführung der Schritte b) und c) aus dem Gemisch das Wasser weitgehend entfernt und

e) die so erhaltenr, weniger als 0,1 Gew.-% Wasser enthaltende Lösung von Wasserstoffperoxid und Orthoborsäure im Lösungsmittel erneut mit dem Olefin umsetzt.

Im einzelnen seien als beispielsweise in das erfindungsgemässe Verfahren einsetzbare Olefine genannt: Ethylen, Propylen, 1-Buten, 2-Buten, Butadien, 1-Penten, 2-Penten, 2-Methyl-1-buten, 2-Methyl-2-buten, Isopren, 2,3-Dimethyl-2-buten, 3,3-Dimethyl-1-buten, 1-Hexen, 2-Hexen, 3-Hexen, 1-Octen, Diisobutylen, 1-Nonen, 1-Decen, Limonen, Pinen, Myrcen, Camphen, 1-Undecen, 1-Dodecen, 1-Tridecen, 1-Tetradecen, 1-Pentadecen, 1-Hexadecen, 1-Heptadecen, 1-Octadecen, Cyclohexen, Cycloocten, Cyclooctadien, Stilben, Vinylcyclohexen, Dicyclopentadien, Sojaöl, Dihydromyrcenol, Safrol, Methylencyclopentan, Methylencyclohexan, 4-Chlor-1-buten und 5,6-Dichlor-1-cycloocten. Es sind auch Oligomere und Polymere der genannten Olefine, soweit sie wenigstens eine Doppelbindung im Moleküle enthalten, im erfindungsgemässen Verfahren einsetzbar, wie beispielsweise Polyisopren, Polybutadien und Polychloropren.

Bevorzugt zur Umsetzung nach dem erfindungsgemässen Verfahren sind Olefine gemäss der vorstehenden Formel (I), worin $R_1$, $R_2$, $R_3$ und $R_4$ unabhängig voneinander Wasserstoff, jedoch mindestens einer der Reste $R_1$ bis $R_4$ $C_1$– bis $C_{30}$– Alkyl, $C_1$– bis $C_{15}$– Alkenyl, $C_3$– bis $C_{12}$– Cycloalkyl, Phenyl oder Naphthyl bedeuten und/oder worin $R_1$ und $R_2$ durch eine Kohlenwasserstoffkette von bis zu 12 Kohlenstoffatomen miteinander verbunden sein können und/oder worin $R_1$ und $R_3$ zusammen mit der zu epoxidierenden Doppelbindung ein 1- bis 3-cyclisches Ringsystem mit bis zu 12

C-Atomen bilden können und worin $R_1$ und $R_2$ gegebenenfalls als Substituenten $C_1$– bis $C_4$– Alkyl, $C_3$– bis $C_6$– Cycloalkyl, $C_1$– bis $C_4$– Alkenyl, Phenyl, $C_1$– $C_4$– Alkoxy, Hydroxy, Fluor oder Chlor enthalten können, wobei zwischen dem Kohlenstoffatom, das als Substituenten Alkoxy, Hydroxy, Carboxy, Fluor oder Chlor enthält, und der zu epoxidierenden Doppelbindung mindestens noch ein Kohlenstoffatom dazwischen steht.

Als Beispiele für derartige Olefine seien im einzelnen genannt:

2-Buten, 2-Penten, 2-Methyl-2-buten, Isopren, 2,3-Dimethyl-2-buten, 2-Hexen, 3-Hexen, Limonen, Pinen, Cyclohexen, Cycloocten, Cyclooctadien, Stilben, Vinylcyclohexen, Dicyclopentadien, Polybutadien, Methylencyclopentan und Methylencyclohexan.

Besonders bevorzugt wird in das erfindungsgemässe Verfahren als Olefin 2,3-Dimethyl-2-buten eingesetzt.

Als Lösungsmittel für das erfindungsgemässe Verfahren sind beispielsweise geeignet:

Alkohole, wie Methanol, Ethanol, n-Propanol, i-Propanol, Butanol, Pentanol, Cyclohexanol, Ethylenglykol, Propylenglykol; Ether, wie Isopropylether, Dioxan, Tetrahydrofuran, Diethylenglykoldimethylether, Diethylenglykoldiethylether; N-substituierte Säureamide, wie Dimethylformamid, sowie N-Methyl-pyrrolidon, Sulfolan und die Ester und Alkylamide von Phosphorsäuren und Phosphonsäuren, wie Triethylphosphat, Tributylphosphat, Trihexylphosphat, Tri-(2-ethyl-hexyl)-phosphat, Trioctylphosphat und Methanphosphonsäuredimethylester. Geeignet sind weiterhin die Phosphorsäurederivate und Phosphonsäurederivate, die in der DE-OS 20 38 319 als Lösungsmittel für Wasserstoffperoxid beschrieben sind.

Bevorzugte Lösungsmittel sind Dioxan, Diethylenglykoldimethylether, Diethylenglykol-diethylether, Tributylphosphat und Methanphosphonsäuredimethylester.

Besonders bevorzugte Lösungsmittel sind Methanphosphonsäuredimethylester und Tributylphosphat.

Verwendet werden können auch Gemische der angegebenen Lösungsmittel.

Der Schritt a) des erfindungsgemässen Verfahrens wird im Temperaturbereich von 30 bis 110 °C durchgeführt. Bevorzugt arbeitet man hier bei 50 bis 105 °C, besonders bevorzugt bei 50 bis 100 °C.

Neben der Arbeitsweise unter isothermen Bedingungen, d.h. Einhaltung einer einheitlichen Temperatur während der gesamten Umsetzung, kann man die Umsetzung auch unter Ausbildung eines sogenannten Temperaturgradienten durchführen, der im allgemeinen mit fortschreitender Reaktion zunimmt. Man kann aber auch die Umsetzung so führen, dass sich mit dem Fortschreiten der Reaktion ein Gradient fallender Temperatur ausbildet.

Die Reaktionszeit für das erfindungsgemässe Verfahren beträgt, je nach der Reaktivität des eingesetzten Olefins zwischen 15 Minuten und 5 Stunden. Die Reaktivitäten einiger Olefintypen sind aus folgender Tabelle ersichtlich (s. Encyclopedia of Polymer Science and Technology, Vol. VI, S. 83, Interscience Publishers, New York 1967), in der Alk für Alkyl und Ar für Aryl steht:

| Olefin | relative Reaktivität | Olefin | relative Reaktivität |
|---|---|---|---|
| $CH_2=CH_2$ | 1 | 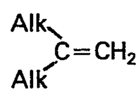 | 500 |
| $H_5C_6CH_2–CH=CH_2$ | 11 | Cyclohexen | 675 |
| Alk– $CH=CH_2$ | 25 | Cyclohepten | 900 |
| Ar–CH=CH–Ar | 27 | Cyclopenten | 1000 |
| Ar–$CH=CH_2$ | 60 | 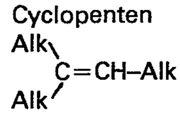 | 6500 |
| Ar–CH=CH–Alk | 240 | 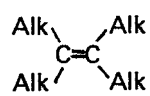 | ≫ 6500 |
| 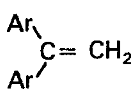 | 250 | | |
| Alk–CH =CH–Alk | 500 | | |

Bei Olefinen mit relativen Reaktivitäten im Bereich 1 bis 250 beträgt die Reaktionszeit im allgemeinen zwischen 1,5 und 5 Stunden, vorzugsweise zwischen 1,8 und 4 Stunden, bei Olefinen mit relativen Reaktivitäten im Bereich über 250 beträgt die Reaktionszeit im allgemeinen 15 Minuten bis 1,5 Stunden, vorzugsweise 30 Minuten bis 1,2 Stunden.

Das Molverhältnis von Olefin zu Wasserstoffperoxid kann beispielsweise 0,1:1 bis 30:1 betragen. Bevorzugt wird ein Molverhältnis von 0,5:1 bis 20:1 angewendet. Besonders vorteilhaft ist es, ein Molverhältnis von Olefin zu Wasserstoffperoxid von 0,8:1 bis 10:1 anzuwenden.

Die erfindungsgemässe Umsetzung kann bei den verschiedensten Drucken durchgeführt werden. Geeignet sind beispielsweise Drucke im Bereich von 1 bis 100 bar. Bevorzugt arbeitet man bei Drucken, die sich bei gegebener Reaktionstemperatur im geschlossenen Gefäss von selbst einstellen.

Das erfindungsgemässe Verfahren wird in Gegenwart der katalytisch wirkenden Orthoborsäure durchgeführt.

Das Gewichtsverhältnis von eingesetzter Menge an Wasserstoffperoxid zur Katalysatormenge kann beispielsweise 1:1 bis 20:1 betragen. Vorzugsweise arbeitet man bei einem Gewichtsverhältnis von 1:1 bis 10:1, besonders bevorzugt bei einem Gewichtsverhältnis von 1:1 bis 5:1.

Der Wasserstoffperoxidgehalt in dem zur Epoxidation verwendeten Lösungsmittel kann beispielsweise 1 bis 30 Gew.–% betragen. Vorzugsweise beträgt der Wasserstoffperoxidgehalt 2 bis 20 Gew.–%. Besonders bevorzugt ist ein Wasserstoffperoxidgehalt von 5 bis 15 Gew.–%.

Der Wassergehalt der zur Epoxidation verwendeten Lösung von Wasserstoffperoxid und Orthoborsäure in einem der angegebenen Lösungsmittel oder Lösungsmittelgemische soll im allgemeinen möglichst niedrig sein. Das erfindungsgemässe Verfahren wird mit einer Wasserstoffperoxid–lösung durchgeführt, die weniger als 0,1 Gew.–% Wasser enthält.

Die Durchführung der erfindungsgemässen Umsetzung kann diskontinuierlich oder kontinuierlich in den für Umsetzungen dieser Art üblichen Vorrichtungen erfolgen. Beispielsweise sind Rührwerkskessel, Siedereaktoren, Röhrenreaktoren, Schlaufenreaktoren und Schleifenreaktoren geeignet.

Als Werkstoffe für die Apparate zur Duchrführung des erfindungsgemässen Verfahrens können verschiedene Materialien verwendet werden. Beispielsweise sind geeignet: Glas, Edelstahl, Nickellegierungen, Zirkon, Tantal oder emailliertes Material.

Die Reaktionswärme kann durch innen- oder aussenliegende Kühler abgeführt werden. Zur Ableitung der Reaktionswärme kann die Umsetzung auch unter Rückfluss, beispielsweise in Siedereaktoren, durchgeführt werden.

Das Olefin kann auf verschiedene Art in die für die Umsetzung verwendete Vorrichtung eingebracht werden. Man kann es gemeinsam mit der Lösung, die das Wasserstoffperoxid und die Orthoborsäure enthält, in der Reaktor geben oder das Olefin und die Lösung getrennt voneinander dem Reaktor zuführen.

Weiterhin ist es möglich, das Olefin und die Wasserstoffperoxid-Lösung zusammen mit der Orthoborsäure an verschiedenen Stellen in die Reaktoreinheit zu leiten. Bei Verwendung mehrerer, in Kaskade geschalteter Reaktoren kann es zweckmässig sein, das gesamte Olefin in den ersten Reaktor einzubringen. Man kann aber auch die Zugabe des Olefins auf die verschiedenen Reaktoren aufteilen.

Aus dem nach der Umsetzung erhaltenen Reaktionsgemisch wird in Schritt b) das entstandene Oxiran und das nichtumgesetzte Olefin abgetrennt. Diese Abtrennung kann gemeinsam oder nacheinander durchgeführt werden, beispielsweise durch Destillation oder Extraktion. Bei dieser Abtrennung kann das während der Reaktion gebildete und ggf. das mit dem Frisch– $H_2O_2$ eingebrachte Wasser oder ein Teil davon mitentfernt werden.

Im Schritt c) wird die Menge Wasserstoffperoxid, die bei der Umsetzung verbraucht worden ist, zugesetzt. Der Schritt c) kann vor, während und/oder nach Schritt b) durchgeführt werden. Das Wasserstoffperoxid kann im allgemeinen in Form einer wässrigen Lösung zugesetzt werden.

In Schritt d) wird aus dem nach Durchführung der Schritte b) und c) vorliegenden Gemisch Wasser abgetrennt, z.B. durch Destillation, Extraktion oder Adsorption. Das abzutrennende Wasser kann aus der zugegebenen wässrigen Wasserstoffperoxidlösung stammen, sowie während der Umsetzung von Olefin und Oxiran gebildetes Wasser sein.

Im Schritt e) wird anschliessend die so erhaltene, weitgehend wasserfreie Lösung von Wasserstoffperoxid und der Orthoborsäure im Lösungsmittel erneut mit dem Olefin umgesetzt.

Im Falle der gemeinsamen Abtrennung von Oxiran, nicht umgesetztem Olefin und gegebenenfalls Reaktionswasser in Schritt b) kann eine Auftrennung dieses Gemisches in seine Komponenten beispielsweise durch Destillation, Extraktion, Absorption, Adsorption oder Extraktivdestillation erfolgen. Das nicht umgesetzte Olefin kann, gegebenenfalls nach vorheriger Reinigung und nach Zugabe der bei der Umsetzung verbrauchten Menge an Olefin, erneut mit der weitgehend wasserfreien Lösung von Wasserstoffperoxid und der Orthoborsäure im Lösungsmittel umgesetzt werden.

Das erfindungsgemässe Verfahren hat gegenüber dem Stand der Technik eine Reihe von Vorteilen. So kann bei niedrigen Reaktionstemperaturen gearbeitet werden, wodurch schnell verlaufende Wasserstoffperoxid-Zersetzungen vermieden werden und aufwendige sicherheitstechnische Massnahmen eingespart werden können. Die Reaktionstemperatur ist innerhalb der beanspruchten Grenzen frei wählbar, da keine Abhängigkeit der Reaktionstemperatur vom Siedepunkt des Olefins vorliegt. Mit dem erfindungsgemässen Verfahren wird ein Gesamtverfahren zur Herstellung von Oxiranen zur Verfügung gestellt, bei dem der Katalysator, nicht umgesetztes Wasserstoffperoxid und nicht umgesetztes Olefin im Kreis geführt und wieder verwendet werden kann. Im übrigen sind nach dem erfindungsgemässen Verfahren eine Vielzahl von Oxiranen herstellbar, die mit hoher Selektivität und Reinheit auf wirtschaftlich vorteilhafte Weise erhalten werden können.

Die nach dem erfindungsgemässen Verfahren erzielbaren guten Ergebnisse sind ausgesprochen überraschend, da gemäss dem Stand der Technik bei Reaktionstemperaturen von 0 bis 120 °C nur dann gute Ergebnisse erhalten werden können, wenn das Reaktionswasser direkt nach dem Entstehen aus dem Reaktionsgemisch entfernt wird. Entfernt man das Reaktionswasser nicht in dem Masse, wie es gebildet wird, aus dem Reaktionsgemisch, so sind gemäss dem Stand der Technik Reaktionstemperaturen von 120 bis 200 °C anzuwenden.

Das erfindungsgemässe Verfahren wird durch folgende Beispiele erläutert, ohne es darauf einzuschränken. Sämtliche Prozentangaben stellen, soweit nichts anderes gesagt wird, Gewichtsprozente dar.

Beispiel 1

168 g/h Tetramethylethylen (2 Mol) werden in einer Rührkesselkaskade, bestehend aus 2 Kesseln, mit 1370,6 g/h einer weitgehend wasserfreien Lösung, bestehend aus 20,6 g Orthoborsäure (0,33 Mol), 68 g Wasserstoffperoxid (2 Mol) und Tributylphosphat bei einer Reaktionstemperatur von 80 °C und einer Verweilzeit von einer Stunde umgesetzt. Dabei wird ein Wasserstoffperoxid-Umsatz von 34,5% erzielt. Tetramethyloxiran wird mit einer Selektivität von 98%, bezogen auf Wasserstoffperoxid, gebildet.

Aus dem Reaktionsgemisch wird anschliessend in einer Destillationseinheit Tetramethylethylen, Tetramethyloxiran und ein Teil des Reaktionswassers abdestilliert. Das Sumpfprodukt aus der Destillation, bestehend aus Wasser, nicht umgesetzten Wasserstoffperoxid und Orthoborsäure, wird mit soviel Wasserstoffperoxid als wässriger Lösung versetzt, wie bei der Reaktion verbraucht worden ist. Das resultierende Gemisch wird in einer zweiten Destillationseinheit vom Wasser befreit und die resultierende, weitgehend wasserfreie Lösung von Orthoborsäure, Wasserstoffperoxid und Tributylphosphat wird in die Reaktionseinheit zurückgeführt und erneut mit Tetramethylethylen umgesetzt.

Das Kopfprodukt aus der ersten Destillationseinheit, bestehend aus Tetramethylethylen, Tetramethyloxiran und Wasser wird in einer dritten Destillationseinheit getrennt. Tetramethylethylen wird zusammen mit dem Wasser als Kopfprodukt erhalten, es wird zusammen mit dem Wasser als Kopfprodukt erhalten, es wird nach Abtrennung des Wassers und Zugabe von soviel Frisch-Tetramethylethylen, wie bei der Reaktion verbraucht wurde, in die Reaktionseinheit zurückgeführt und mit der weitgehend wasserfreien Lösung von Wasserstoffperoxid, Orthoborsäure und Tributylphosphat umgesetzt. Das in der dritten Destillationseinheit als Sumpfprodukt erhaltene Tetramethyloxiran wird in einer vierten Destillationseinheit als Kopfprodukt erhalten.

Beispiel 2

In einem Autoklaven werden 210 g 3,3-Dimethyl-1-buten (2,5 Mol) vorgelegt und mit 34 g Orthoborsäure (0,55 Mol) sowie 1750 g einer nahezu wasserfreien, 4,8 Gew.-%-igen $H_2O_2$-Lösung in Methanphosphonsäuredimethylester (2,5 Mol) versetzt, mit 10 bar Stickstoff überlagert und 3 Stunden auf 110 °C erwärmt. In dem Reaktionsgemisch werden gaschromatographisch 0,58 Mol t-Butyloxiran sowie jodometrisch 1,95 Mol Wasserstoffperoxid bestimmt. Dies entspricht einem Umsatz von 26% und einer Selektivität von 89% bezüglich des Wasserstoffperoxids.

Die Aufarbeitung des Reaktionsgemisches erfolgte wie in Beispiel 1 beschrieben.

Beispiel 3

In einem Autoklaven wurden 216 g einer nahezu wasserfreien, 11,2 Gew.–%-igen $H_2O_2$-Lösung in Tributylphosphat (0,71 Mol) vorgelegt, mit 4 g Orthoborsäure (0,06 Mol) und 194 g Propylen (4,64 Mol) versetzt, mit 10 bar Stickstoff überlagert und 2 Stunden auf 80 °C erhitzt.

In dem Reaktionsgemisch wurden gaschromatographisch 0,11 Mol Propylenoxid sowie jodometrisch 0,56 Mol Wasserstoffperoxid bestimmt.

Die Aufarbeitung des Reaktionsgemisches erfolgte wie in Beispiel 1 beschrieben.

**Patentansprüche:**

1) Verfahren zur Herstellung von Oxiranen aus Olefinen und Wasserstoffperoxid in Gegenwart von Orthoborsäure, dadurch gekennzeichnet, dass man

a) ein Olefin der allgemeinen Formel

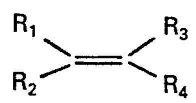

(I),

worin

$R_1$, $R_2$, $R_3$ und $R_4$ unabhängig voneinander Wasserstoff, $C_1$– bis $C_{30}$-Alkyl, $C_1$– bis $C_{15}$-Alkenyl, $C_3$-$C_{12}$- Cycloalkyl, Phenyl oder Naphthyl bedeuten und/oder worin $R_1$ und $R_2$ durch eine Kohlenwasserstoffkette von bis zu 12 Kohlenstoffatomen miteinander verbunden sein können und/oder worin $R_1$ mit $R_3$ zusammen mit der zu epoxidierenden Doppelbindung ein 1– bis 3-cyclisches Ringsystem mit bis zu 12 Kohlenstoffatomen bilden können und worin $R_1$, $R_2$, $R_3$ und $R_4$ gegebenenfalls als Substituenten $C_1$– bis $C_4$-Alkyl, $C_3$– bis $C_6$-Cycloalkyl, $C_1$– bis $C_4$– Alkenyl, Phenyl, $C_1$– bis $C_4$-Alkoxy, Hydroxy, Carboxy oder Halogen enthalten können, wobei zwischen dem Kohlenstoffatom, dass als Substituenten Alkoxy, Hydroxy, Carboxy oder Halogen enthält, und der zu epoxidierenden Doppelbindung mindestens noch ein Kohlenstoffatom dazwischen steht, mit einer weniger als 0,1 Gew.-% Wasser enthaltenden Lösung von Wasserstoffperoxid in einem Lösungsmittel bei einer Temperatur 30 bis 110 °C und bei einer Reaktionszeit, die je nach Reaktivität des eingesetzten Olefins zwischen 15 Minuten und 5 Stunden beträgt, umsetzt,

b) das entstandene Oxiran und das nicht umgesetzte Olefin abtrennt,

c) vor, während und/oder nach der Abtrennung von Oxiran und Olefin der Reaktionsmischung die Menge Wasserstoffperoxid zusetzt, die bei der Reaktion verbraucht worden ist,

d) nach Durchführung der Schritte b) und c) aus dem Gemisch das Wasser weitgehend entfernt und

e) die so erhaltene, weniger als 0,1 Gew.-% Wasser enthaltende Lösung von Wasserstoffperoxid und Orthoborsäure im Lösungsmittel erneut mit dem Olefin umsetzt.

2) Verfahren nach Anspruch 1, dadurch gekennzeichnet, dass man als Olefin eine Verbindung aus der Gruppe 2-Buten, 2-Penten, 2-Methyl-2-buten, Isopren, 2,3-Dimethyl-2-buten, 2-Hexen, 3-Hexen, Limonen, Pinen, Cyclohexen, Cycloocten, Cyclooctadien, Stilben, Vinylcyclohexen, Dicyclopentadien, Polybutadien, Methylencyclopentan und Methylencyclohexan einsetzt.

3) Verfahren anch Anspruch 1 und 2, dadurch gekennzeichnet, dass man als Lösungsmittel Alkohole, Ether, N-substituierte Säureamide, N-Methylpyrrolidon, Sulfolan und/oder Ester und/oder Alkylamide von Phosphorsäuren oder Phosphonsäuren oder Gemische dieser Lösungsmittel einsetzt.

4) Verfahren nach Anspruch 1 bis 3, dadurch gekennzeichnet, dass das Molverhältnis von Olefin zu Wasserstoffperoxid 0,1:1 bis 30:1 beträgt.

5) Verfahren nach Anspruch 1 bis 4, dadurch gekennzeichnet, dass die Umsetzung bei Drucken im Bereich von 1 bis 100 bar durchgeführt wird.

6) Verfahren nach Anspruch 1 bis 5, dadurch gekennzeichnet, dass das Gewichtsverhältnis von eingesetzter Menge an Wasserstoffperoxid zur Menge an Orthoborsäure 1:1 bis 20:1 beträgt.

7) Verfahren nach Anspruch 1 bis 6, dadurch gekennzeichnet, dass in Schritt b) die Abtrennung von gebildetem Oxiran und nicht umgesetzten Olefinen gemeinsam oder nacheinander durch Destillation oder Extraktion durchgeführt wird.

8) Verfahren nach Anspruch 1, dadurch gekennzeichnet, dass in Schritt b) das während der Reaktion gebildete Wasser ganz oder teilweise mitentfernt wird.

9) Verfahren nach Anspruch 1 bis 8, dadurch gekennzeichnet, dass man Oxiran, nicht umgesetztes Olefin und gegebenenfalls Reaktionswasser in Schritt b) gemeinsam abtrennt und dieses Gemisch in seine Komponeneten auftrennt durch Destillation, Extraktion, Absorption, Adsorption oder Extraktivdestillation.

10) Verfahren nach Anspruch 1 bis 9, dadurch gekennzeichnet, dass man das in Schritt b) abgetrennte, nicht umgesetzte Olefin nach vorheriger Reinigung und nach Zugabe der bei der Reaktion verbrauchten Menge an Olefin erneut umsetzt.

**Revendications**

1. Procédé de fabrication d'oxiranes à partir d'oléfines et de peroxyde d'hydrogène en présence d'acide orthoborique, caractérisé en ce qu'on fait réagir

a) une oléfine de formule générale:

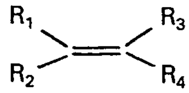

(I),

dans laquelle

$R_1$, $R_2$, $R_3$ et $R_4$ indépendamment les uns des autres représentent de l'hydrogène, alcoyle en $C_1$ à $C_{30}$, alcényle en $C_1$ à $C_{15}$, cycloalcoyle en $C_3$ à $C_{12}$, phényle ou naphtyle et/ou dans laquelle $R_1$ et $R_2$ peuvent être reliés entre eux par une chaîne hydrocarbonée ayant jusqu'à 12 atomes de carbone, et/ou dans laquelle $R_1$ avec $R_3$, conjointement avec la double liaison à époxyder, peuvent former un système nucléaire comportant 1 à 3 cycles renfermant jusqu'à 12 atomes de carbone, et dans laquelle $R_1$ $R_2$, $R_3$ et $R_4$ peuvent éventuellement contenir comme substituants un alcoyle en $C_1$-$C_4$, cycloalcoyle en $C_3$ à $C_6$, alcényle en $C_1$ à $C_4$, phényle, alcoxy en $C_1$-$C_4$, hydroxy, carboxy ou de l'halogène, cas où, entre l'atome de carbone qui contient comme substituants un alcoxy, hydroxy, carboxy ou de l'halogène et la double liaison à époxyder, se trouve entre eux au moins encore un atome de carbone, avec une solution, contenant moins de 0,1% en poids d'eau, de peroxyde d'hydrogène dans un solvant à une température de 30 à 110 °C et pour un temps de réaction qui, suivant la réactivité de l'oléfine employée, s'élève à 15 minutes à 5 heures,

b) on sépare l'oxirane obtenu et l'oléfine qui n'a pas réagi,

c) avant, pendant et/ou après la séparation de l'oxirane et de l'oléfine on ajoute au mélange de réaction la quantité de peroxyde d'hydrogène qui à été consommé dans la réaction,

d) après exécution des stades b) et c) on élimine largement l'eau à partir du mélange et

e) on fait réagir la solution obtenue, contenant moins da 0,2% en poids d'eau, du peroxyde d'hydrogène et de l'acide orthoborique dans le solvant, de nouveau avec l'oléfine.

2. Procédé selon la revendication 1, caractérisé en ce qu'on utilise comme oléfine un composé du groupe du 2-butène, 2-pentène, 2-méthyl-2-butène, isoprène, 2,3-diméthyl-2-butène, 2-hexène, 3-hexène, limonène, pinène, cyclohexène, cyclooctène, cyclooctadiène, stilbène, vinylcyclohexène, dicyclopentadiène, polybutadiène, méthylènecyclopentane et méthylènecyclohexane.

3. Procédé selon les revendications 1 et 2, caractérisé en ce qu'on utilise comme solvant des alcools, des éthers, des amides d'acides N-substituées, de la N-méthylpyrrolidone, du sulfolane et/ou des esters et/ou alcoylamides d'acides phosphoriques ou d'acides phosphoniques, ou des mélanges de ces solvants.

4. Procédé selon les revendications 1 à 3, caractérisé en ce que le rapport molaire de l'oléfine au peroxyde d'hydrogène est de 0,1:1 jusqu'à 30:1.

5. Procédé selon les revendications 1 à 4, caractérisé en ce qu'on exécute la réaction sous des pressions dans l'intervalle de 1 à 100 bars.

6. Procédé selon les revendications 1 à 5, caractérisé en ce que le rapport pondéral de la quantité

ajoutée de peroxyde d'hydrogène à la quantité d'acide orthoborique est de 1:1 jusqu'à 20:1.

7. Procédé selon les revendications 1 à 6, caractérisé en ce que dans le stade b) on exécute la séparation de l'oxirane formé et de l'oléfine qui n'a pas réagi conjointement ou successivement, par distillation ou extraction.

8. Procédé selon la revendication 7, caractérisé en ce que dans le stade b) on élimine conjointement, en totalité ou en partie, l'eau qui s'est formée pendant la réaction.

9. Procédé selon les revendications 1 à 8, caractérisé en ce qu'on sépare en commun au stade b) l'oxirane, l'oléfine qui n'a pas réagi et éventuellement l'eau de réaction et l'on sépare ce mélange en ses composants par distillation, extraction, absorption, adsortion ou distillation extractive.

10. Procédé selon les revendications 1 à 9, caractérisé en ce que l'oléfine qui n'a pas réagi et qui a été séparée au stade b) est de nouveau mise à réagir, après purification préalable et après addition de la quantité d'oléfine consommée dans la réaction.

**Claims:**

1) Process for the preparation of oxiranes from olefines and hydrogen peroxide in the presence of orthoboric acid, characterised in that

a) an olefine of the general formula

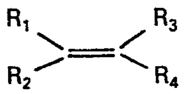

(I),

wherein

$R_1$, $R_2$, $R_3$ and $R_4$ independently of one another denote hydrogen, $C_1$- to $C_{30}$-alkyl, $C_1$-to $C_{15}$-alkenyl, $C_3$-$C_{11}$-cycloalkyl, phenyl or naphthyl, and/or wherein

$R_1$ and $R_2$ can be linked to one another by a hydrocarbon chain with up to 12 carbon atoms, and/or wherein

$R_1$ and $R_3$, together with the double bond to be epoxidised can form a mono- di- or tri-cyclic ring system with up to 12 carbon atoms, and wherein $R_1$, $R_2$, $R_3$ and $R_4$ can optionally contain as substituents $C_1$- to $C_4$-alkyl, $C_3$- to $C_6$-cycloalkyl, $C_1$- to $C_4$-alkenyl, phenyl, $C_1$- to $C_4$-alkoxy, hydroxyl, carboxyl or halogen, there being at least one further carbon atom between the carbon atom which contains alkoxy, hydroxyl, carboxyl or halogen as a substituent and the double bond to be epoxidised, is reacted with a solution, containing less than 0,1% by weight of water, of hydrogen peroxide in a solvent at a temperature of 30 to 110 °C and for a reaction time which, depending on the reactivity of the olefin introduced, is between 15 minutes and 5 hours,

b) the oxirane formed and the unreacted olefine are separated off,

c) the amount of hydrogen peroxide which has been consumed during the reaction is added to the reaction mixture before, during and/or after separating off the oxirane and olefine,

d) after carrying out steps b) and c), the water is largely removed from the reaction mixture and

e) the resulting solution, containing less than 0.1% by weight of water, of hydrogen peroxide and orthoboric acid in the solvent is reacted again with the olefine.

2) Process according to Claim 1, characterised in that a compound from the group comprising 2-butene, 2-pentene, 2-methyl-2-butene, isoprene, 2,3-dimethyl-2-butene, 2-hexene, 3-hexene, limonene, pinene, cyclohexene, cyclooctene, cyclooctadiene, stilbene, vinylcyclohexene, dicyclopentadiene, polybutadiene, methylenecyclopentane and methylenecyclohexane is employed as the olefine.

3) Process according to Claims 1 and 2, characterised in that alcohols, ethers, N-substituted acid amides, N-methylpyrrolidone, sulpholane and/or esters and/or alkylamides of phosphoric acids or phosphonic acids or mixtures of these solvents, are employed as the solvent.

4) Process according to Claims 1 to 3, characterised in that the molar ratio of olefine to hydrogen peroxide is 0,1:1 to 30:1.

5) Process according to Claims 1 to 4, characterised in that the reaction is carried out under pressures in the range from 1 to 100 bars.

6) Process according to Claims 1 to 5, characterised in that the weight ratio of the amount of hydrogen peroxide employed to the amount of orthoboric acid is 1:1 to 20:1.

7) Process according to Claims 1 to 6, characterised in that, in step b), the oxirane formed and the unreacted olefine are separated off together or successively, by distillation or extraction.

8) Process according to Claim 7, characterised in that all or some of the water formed during the reaction is also removed in step b).

9) Process according to Claims 1 to 8, characterised in that the oxirane, unreacted olefine and, if appropriate, water of reaction are separated off together in step b) and this mixture is separated into its components by distillation, extraction, absorption, adsorption or extractive distillation

10) Process according to Claims 1 to 9, characterised in that the unreacted olefine separated off in step b) is reacted again, after prior purification and after adding the amount of olefine consumed in the reaction.